(19) Europäisches Patentamt

European Patent Office

Office européen des brevets

(11) **EP 1 121 144 B1**

(12) **EUROPEAN PATENT SPECIFICATION**

(45) Date of publication and mention
of the grant of the patent:
**05.06.2002   Bulletin 2002/23**

(51) Int Cl.$^{7}$: **A61K 38/28**

(86) International application number:
**PCT/DK99/00556**

(21) Application number: **99948725.9**

(22) Date of filing: **15.10.1999**

(87) International publication number:
**WO 00/23098 (27.04.2000 Gazette 2000/17)**

(54) **STABLE CONCENTRATED INSULIN PREPARATIONS FOR PULMONARY DELIVERY**

STABILE KONZENTRIERTE INSULIN PRÄPARATIONEN ZUR PULMONAREN VERABREICHUNG

PREPARATIONS D'INSULINE, CONCENTREES ET STABLES, DESTINEES A L'ADMINISTRATION PAR VOIE PULMONAIRE

(84) Designated Contracting States:
**AT BE CH CY DE DK ES FI FR GB GR IE IT LI LU MC NL PT SE**
Designated Extension States:
**LT RO SI**

(30) Priority: **16.10.1998   DK 132798**

(43) Date of publication of application:
**08.08.2001   Bulletin 2001/32**

(60) Divisional application:
**01126054.4 / 1 172 114**

(73) Proprietor: **Novo Nordisk A/S
2880 Bagsvaerd (DK)**

(72) Inventor: **HAVELUND, Svend
DK-2880 Bagsvaerd (DK)**

(74) Representative: **Kellberg, Lars
Novo Nordisk A/S,
Corporate Patents,
Novo Allé
2880 Bagsvaerd (DK)**

(56) References cited:
**EP-A1- 0 692 489        WO-A1-97/48413
WO-A1-98/42367        WO-A1-98/42368
US-A- 5 474 978        US-A- 5 506 203**

## Description

### Field of the invention

The present invention relates to concentrated aqueous insulin formulations of high physical and chemical stability and being suitable for pulmonary delivery.

### Background of the invention

Diabetes is a general term for disorders in man having excessive urine excretion as in diabetes mellitus and diabetes insipidus. Diabetes mellitus is a metabolic disorder in which the ability to utilize glucose is more or less completely lost. About 2 % of all people suffer from diabetes.

Since the introduction of insulin in the 1920's, continuous strides have been made to improve the treatment of diabetes mellitus. To help avoid extreme glycaemia levels, diabetic patients often practice multiple injection therapy, whereby insulin is administered with each meal.

In solution, the self-association pattern of insulin is a complex function of protein concentration, metal ions, pH, ionic strength and solvent composition. For the currently used soluble preparations containing U100 insulin, zinc ions, isotonic agent and phenolic preservative, the following equilibria must be considered:

$$6 \text{ In} \leftrightarrow 3 \text{ In}_2$$

$$3 \text{ In}_2 + 2 \text{ Zn}^{2+} \leftrightarrow \text{In}_6 \text{ (T}_6\text{)}$$

$$T_6 \leftrightarrow T_3R_3 \leftrightarrow R_6$$

The known degradation patterns of insulin include a) fibril formation; b) deamidations at A18, A21 and B3; c) dimerisations via transamidation or Schiff-base formation; d) disulfide exchange reactions.

According to Brange (Stability of Insulin, Kluwer Academic Press, 1994), each of these degradation reactions proceed much faster in the monomeric state than in the hexameric state. Therefore, the most efficient means of stabilising insulin preparations is by pushing the above equilibrium as far to the right as possible. In addition to this general effect of mass action, the reactivity of selected residues is further modified depending on their direct involvement in the T $\rightarrow$ R conformational change. Thus, the reactivity of B3Asn is much lower in the R-state (when the residue resides in an $\alpha$-helix) than in the T-state.

The interconversion between $T_6$, $T_3R_3$ and $R_6$ conformations of the two zinc insulin hexamer is modulated by ligand binding to the $T_3R_3$ and $R_6$ forms. Anions such as chloride have affinity for the fourth coordination position in the metal ions of $T_3R_3$ and $R_6$, while preservatives such as phenol binds to hydrophobic pockets located near the surfaces of the $T_3R_3$ and $R_6$ forms (Derewenda, Nature 338, 594, 1989 and, Brzovic, Biochemistry 33, 130557, 1994). By the use of Co$^{2+}$ insulin it has been shown that the combined effect of anion and phenol binding is particularly efficient in stabilising the R$_6$ state. (Brader, Trends Biochem. Sci. 30, 6636, 1991 and; Bloom, J. Mol. Biol. 245, 324, 1995). Furthermore, for both Zn$^{2+}$- and Co$^{2+}$ insulin it has been shown that phenol is much more efficient than m-cresol in inducing R-state in the insulin hexamer (Wollmer, Biol. Chem. Hoppe-Seyler 368, 903, 1987 and, Choi, Biochemistry 32, 11638, 1993). High affinity phenol derivatives inducing R-state are 7-hydroxy-indol ((Dodson, Phil. Trans. R. Soc. Lond. A 345, 153, 1993) resorcinol and 2,6- and 2,7-dihydroxy-naphtalen ((Bloom, J. Mol. Biol. 245, 324, 1995). The physical denaturation of insulin is known as fibrillation. In the fibrillar state extended peptide chains are laying parallel or anti parallel and hydrogen bonded to each other, so-called $\beta$-structure or $\beta$-pleated sheets. Fibrils represent usually the lowest state of energy of the protein, and only harsh conditions such as strong base may enable a regeneration from this state to the native state of correctly folded protein. Factors that promote the rate of formation of fibrils are increasing the temperature, increasing the surface area between the liquid and the air phase and, for zinc-free insulin, increasing the concentration. For hexameric zinc-insulin the rate of fibril formation decreases with increasing concentration. The formation of fibrils is believed to proceed via monomerization of insulin. Fibrils of insulin have the appearance of gels or precipitates.

Insulin derivatives having truncations in the C-terminal of the B-chain, e.g. despentapeptide (B26-B30) insulin and des-octapeptide (B23-B30) insulin are more prone to form fibrils than human insulin. Insulin analogues which dissociate readily from the hexameric unit to the monomeric form, e.g. the AspB28 human insulin and the LysB28-ProB29 human insulin, are likewise more prone to form fibrils than human insulin.

The native state of insulin is stabilised by bringing about the conditions that stabilises the hexameric unit, i. e. the presence of zinc ions (2-4 zinc/hexamer), phenol (0.1-0.5% w/v) and sodium chloride (5-150 mM).

Addition of agents that reduce the surface tension at the air-liquid interface further reduces the propensity to fibril formation. Thus, polyethylene glycol, polypropylene glycol and copolymers hereof with an average molecular weights of about 1800 have found use as stabilisers in concentrated insulin solutions for infusion pumps (Grau, 1982. In: Neue Insuline (Eds. Petersen, Schlüter & Kerp), Freiburger Graphische Betriebe, pp. 411-419 and Thurow,1981: patent DE2952119A1). For a comprehensive review on the physical stability of insulin see Brange 1994, Stability of Insulin, Kluwer Academic Publisher, pp. 18-23. Most of the chemical degradation of insulin in preparations is due to reactions involving the carboxamide function of the asparagine residues, in particular residues B3 and A21. Hydrolysis of the amide groups leads to desamido derivatives, and transamidation involving an amino group from another molecule leads to covalently linked dimers and, after similar consecutive reactions, to trimers and higher polymers.

In acid solution AsnA21 is the most reactive, leading to AspA21 insulin (Sundby, J. Biol. Chem. 237, 3406, 1962). In crude insulin of bovine and porcine origin, obtained by acid ethanol extraction, the most abundant dimers isolated were AspA21 -GlyA1 and AspA21 - PheB1 linked (Helbig 1976, Insulindimere aus der B-Komponente von Insulinpraparationen, Thesis at the Rheinisch-Westfälischen Technischen Hochschule, Aachen).

In neutral solution, which is the preferred embodiment of insulin preparations for injection therapy, AsnB3 is the most susceptible residue. Degradation products include AspB3 insulin, AspB3 -GlnB4 isopeptide insulin, and dimers and higher polymers where AspB3 provides the carbonyl moiety of a peptide bond with an amino group of another molecule. For a comprehensive review on the chemical stability of insulin see Brange 1994, Stability of Insulin, Kluwer Academic Publisher, pp. 23-36. As for the physical stability conditions that stabilises the hexameric unit, i.e. the presence of zinc ions (2-4 zinc/hexamer), phenol (0.1-0.5% w/v) and sodium chloride (5-150 mM), decrease the rate of formation of degradation products during storage at neutral pH.

A different type of polymerisation reaction is observed when the conditions that stabilises the hexameric unit is neglected. Thus, in the absence of zinc, phenol and sodium chloride, and using a temperature of 50°C, disulfide-linked dimers and high molecular weight polymers are the prevailing products formed. The mechanism of formation is a disulfide interchange reaction, resulting from $\beta$-elimination of the disulfides (Brems, Protein Engineering 5, 519, 1992).

Solubility of insulin is a function of pH, metal ion concentration, ion strength, phenolic substances, solvent composition (polyols, ethanol and other solvents), purity, and species (bovine, porcine, human, other analogues). For a review see Brange: Galenics of Insulin, Springer-Verlag 1987, p.18 and 46.

The solubility of insulin is low at pH values near its isoelectric pH, i.e. in the pH range 4.0 - 7.0. Highly concentrated solutions of porcine insulin (5000 U/ml $\sim$ 30 mM) have been brought about at acid pH (Galloway, Diabetes Care 4, 366, 1981), but the insulin in the formulation is highly instable due to deamidation at AsnA21. At neutral pH highly concentrated solutions of zinc free insulin can be made, but these are unstable due to a high rate of polymerisation and deamidation at AsnB3. Porcine zinc insulin solutions at neutral pH comprising phenol have been reported physical stable at concentrations of 1000 U/ml at elevated temperature, but become supersaturated when the temperature is lowered to 4 °C. (Brange and Havelund in Artificial Systems for Insulin Delivery, Brunetti et al. eds, Raven Press 1983).

In order to reduce the inconvenience of insulin injections much attention has been given to alternative routes of administration (for an overview see Brange and Langkjær in Protein Delivery: Physical Systems, Sanders and Hendren, eds., Plenum Press 1997). Pulmonary delivery seems to be the most promising of these (Service, Science 277,1199.1997). Insulin can be given aerolised in the form of dry powder or as nebulised droplets from an insulin solution. The efficacy might be enhanced by coached breathing (Gonda, US Patent 5,743,250) and addition of an absorption enhancer (Baekstroem, US Patent 5,747,445) or protease inhibitors (Okumura, Int. J. Pharm. 88, 63, 1992).

The bioavailability of a nebulised concentrated insulin solution (500 U/ml) was shown to be 20-25 % as compared to a subcutaneous injection (Elliot, Aust. Paediatr. J. 23, 293, 1987). By using 30-50 μl insulin solution per puff the insulin solution need to be 5-20 times more concentrated than the usual concentration of 0.6 mM. By using a single dose container, e.g. a blister pack (Gonda, US Patent 5,743,250), the demand for a preservative is abolished. Most insulin formulations are preserved by the toxic, mucose irritating and unpleasant odorous phenol and m-cresol. However, omitting phenols will cause stability problems. In addition to the bacteriostatic efficacy, the phenols act as physico-chemical stabilisers of insulin in combination with zinc ions. So, it is preferred that formulations of insulin for inhalation are made with a minimum concentration of phenol or that phenol has been replaced by more acceptable substitutes.

**Description of the invention**

Definitions

By "analogue of human insulin" (and similar expressions) as used herein is meant human insulin in which one or more amino acids have been deleted and/or replaced by other amino acids, including non-codeable amino acids, or human insulin comprising additional amino acids, i.e. more than 51 amino acids.

By "derivative of human insulin" (and similar expressions) as used herein is meant human insulin or an analogue thereof in which at least one organic substituent is bound to one or more of the amino acids.

By "phenolic molecule" as used herein is meant phenol or any derivative thereof such as m-cresol or chlorocresol.

Brief description of the invention

It is an object of the present invention to provide a concentrated insulin formulation for pulmonary delivery having an acceptable physical and chemical stability.

This object has unexpectedly been accomplished by providing an insulin formulation in which the concentration of chloride is kept below 50 mM, and in which the concentration of other anions such as phosphate is minimised.

Accordingly, the present invention relates to an aqueous insulin formulation comprising: 6 to 15 mM of human insulin or an analogue or a derivative thereof, less than 50 mM of chloride, less than 10 mM of any anions other than chloride and acetate, 2 to 5 $Zn^{2+}$ ions per six molecules of insulin, and at least 3 phenolic molecules per six molecules of insulin.

Preferred embodiments

In certain advantageous embodiments, the formulation of the invention comprises about 6 mM, about 9 mM, about 12 mM, or about 15 mM of human insulin or an analogue or a derivative thereof.

When the insulin formulation of the invention is to be administered from multi-dose containers a preservative effect is desired and it may thus advantageously contain up to 50 mM of phenolic molecules. Surprisingly, however, adequate stability is obtained by using a relatively low concentration of phenolic molecules such as 3 to 12 phenolic molecules per six molecules of insulin, preferably 3 to 9 phenolic molecules per six molecules of insulin. A low concentration of phenolic molecules can be used when no or little preservative action is needed such as in single-dose containers. A further advantage of using a low amount of phenolic molecules is an increased convenience for the patient.

The insulin formulation according to the invention preferably contain less than 40 mM, more preferably less than 30 mM of chloride, still more preferably 5 to 20 mM of chloride, in order to secure optimal stability.

In a particular embodiment the insulin may comprise a low amount of phosphate buffer, preferably up to 5 mM of phosphate.

Insulin formulations of the invention comprising 2 to 4 $Zn^{2+}$ ions, preferably 2.2 to 3.2 $Zn^{2+}$ ions per six molecules of insulin, are very stable.

Insulin formulations of the invention comprising 3 to 5 $Zn^{2+}$ ions, preferably 3.5 to 5 $Zn^{2+}$ ions per six molecules of insulin, are also suitable.

Surprisingly, it is possible to add relatively high concentrations of zwitterions such as glycylglycine and glycine to the insulin formulation of the invention without decreasing the solubility of insulin. Glycylglycine acts as a buffer at neutral pH and furthermore increase the dissolution rate of zinc insulin at neutral to basic pH due to a moderately zinc chelating effect. Also, glycylglycine may act as a scavenger for amine reactions during the storage period. Thus, in a preferred embodiment the insulin formulation of the invention further comprises 5 to 150 mM of a zwitterionic amine, preferably glycylglycine or glycine.

In a preferred embodiment the insulin formulation of the invention further comprises 5 to 50 mM of trishydroxymethylaminomethan which acts as a buffer at neutral pH and as a scavenger for amine reactive compounds.

In another preferred embodiment the insulin formulation of the invention comprises sodium ions as cations. The sodium ion has a low salting out effect.

In another preferred embodiment the insulin formulation of the invention comprises potassium or a mixture of potassium and sodium ions as cations. Potassium ions in a concentration higher than the plasma concentration of 4-5 mM increase the transport of insulin through the lungs.

In another preferred embodiment potassium ion in a concentration more than 4-5 mM is used in combination with a mild bronchodilator such as menthol.

In another preferred embodiment the insulin formulation of the invention comprises between 0.001 % by weight and 1 % by weight of a non-ionic surfactant, preferably tween 20 or Polox 188. A nonionic detergent can be added to stabilise insulin against fibrillation during storage and nebulisation.

In another preferred embodiment the insulin formulation of the invention comprises 1 mM to 10 mM of an anionic surfactant, preferably sodium taurocholate, in order to further increase the bioavailabilty of insulin.

In a preferred embodiment the insulin used is human insulin.

In another preferred embodiment the insulin used is an analogue of human insulin wherein position B28 is Asp, Lys, Leu, Val or Ala and position B29 is Lys or Pro; or des(B28-B30), des(B27) or des(B30) human insulin.

The preferred analogues of human insulin are those in which position B28 is Asp or Lys, and position B29 is Lys or Pro, preferably Asp$^{B28}$ human insulin or Lys$^{B28}$Pro$^{B29}$ human insulin.

In another preferred embodiment the insulin is selected from the group of soluble long-acting insulin derivatives such as derivatives of human insulin having one or more lipophilic substituents, preferably acylated insulins.

The insulin derivative according to this embodiment is preferably selected from the group consisting of B29-N$^{\varepsilon}$-myristoyl-des(B30) human insulin, B29-N$^{\varepsilon}$-palmitoyl-des(B30) human insulin, B29-N$^{\varepsilon}$-myristoyl human insulin, B29-N$^{\varepsilon}$-palmitoyl human insulin, B28-N$^{\varepsilon}$-myristoyl Lys$^{B28}$ Pro$^{B29}$ human insulin, B28-N$^{\varepsilon}$-palmitoyl Lys$^{B28}$ Pro$^{B29}$ human insulin, B30-N$^{\varepsilon}$-myristoyl-Thr$^{B29}$Lys$^{B30}$ human insulin, B30-N$^{\varepsilon}$-palmitoyl-Thr$^{B29}$Lys$^{B30}$ human insulin, B29-N$^{\varepsilon}$-(N-palmitoyl-$\gamma$-glutamyl)-des(B30) human insulin, B29-N$^{\varepsilon}$-(N-lithocholyl-$\gamma$-glutamyl)-des(B30) human insulin, B29-N$^{\varepsilon}$-($\omega$-carboxyheptadecanoyl)-des(B30) human insulin and B29-N$^{\varepsilon}$-($\omega$-carboxyheptadecanoyl) human insulin.

The most preferred insulin derivative is B29-N$^{\varepsilon}$-myristoyl-des(B30) human insulin or B29-N$^{\varepsilon}$-(N-lithocholyl-$\gamma$-glutamyl)-des(B30) human insulin.

The above soluble long acting insulin derivatives are albumin binding and have been designed to provide a constant basal supply of insulin (Markussen, Diabetologia 39, 281, 1996). Subcutaneous administration once or twice daily secures the required basal delivery of insulin, whereas for pulmonary administration several daily inhalations are recommended, preferably in connection with meals.

The insulin derivatives have a protracted onset of action and may thus compensate the very rapid increase in plasma insulin normally associated with pulmonary administration. By careful selection of the type of insulin, the present invention enables adjustment of the timing, and in order to obtain the desired insulin profile.

In a particular embodiment of the present invention, the insulin formulation comprises an insulin analogue or human insulin as well as an insulin derivative.

The phenolic molecules in the insulin formulation are preferably selected from the group consisting of phenol, m-cresol, chloro-cresol, thymol, or any mixture thereof.

The insulin preparation of the present invention preferably has a pH value in the range of 7 to 8.5, more preferably 7.4 to 7.9.

This invention is further illustrated by the following examples which, however, are not to be construed as limiting.

EXAMPLE 1

2.5 ml of a 21 mM insulin stock solution was made by dissolving 337 mg zinc free human insulin in 1237 μl water and adding 263 μl of 0.1 M ZnCl$_2$ and 637 μl water before adjusting pH with 38 μl of 0.2 M NaOH and finally adding water to 2.5 ml, calculating the specific volume of insulin as 0.7 μl/mg. A preparation of 15 mM was then made by adding 350 μl of 0.16 M m-cresol, 175 μl of 0.32 M phenol and salt or detergent to the concentrations shown in Table 1 and thereafter diluted by medium to 12, 9, 6, 3 and 0.6 mM and stored at 5 °C.

EXAMPLE 2

Zinc insulin was dispersed in water (1:10) on icebath, added glycylglycine (7/15) equivalent and sodium hydroxide (3.1 equivalent) and stirred slowly overnight at 5 °C. 0.1 equivalent of zinc chloride and detergent was then added, pH adjusted to 7.5 by 0.8 equivalent of hydrochloric acid and volume adjusted before adding phenol and water and finally diluting the 15 mM preparation with medium containing sodium chloride, glycylglycine and detergent to obtain 12, 9, 6, and 3 mM of human insulin. (Table 2 and 3).

The results are presented in the following Tables 1 to 3.

The data of Table 1 show that even a small amount of phosphate (e.g. 5 mM) reduce the stability of insulin, and substituting sodium chloride by trihydroxymethylaminomethan hydrochloride also tends to decrease the solubility of insulin. Contrary to salts the zwitterions glycylglycine and glycine increased the solubility of insulin, and it was possible to add unexpectedly high concentrations of the zwitterions glycylglycine and glycine without deteriorating the stabilising effect on insulin. Glycylglycine acts as a buffer at neutral pH and furthermore increases the dissolution rate of zinc insulin at neutral to basic pH due to a moderately zinc chelating effect. Glycylglycine may also act as a scavenger for amine reactions during storage. Addition of the non-ionic detergents tween 20 and poloxamer 188 up to 1 % by weight and 3 mM of the anionic detergent sodium taurocholate did not reduce the stability at 5°C storage.

An evaluation of the effect of a phenolic substance added equimolar to insulin is shown in table 2. Three of the phenolic molecules increase the physical stability from 6 to 15 mM of insulin or more at low temperature and reduce the formation of polymers at elevated temperature by a factor of 2 - 3 (at low chloride concentration). In another set of experiments (table 3) the relative amount of phenol or chloro-cresol is varied from 0 to 2 per insulin at increasing chemical stability.

EXAMPLE 3

441 mg B29-N$^\varepsilon$-(N-lithocholyl-$\gamma$-glutamyl)-des(B30) human insulin (143 nmol/mg) was suspended in 5 ml water at 0 °C and 220 $\mu$l 1 N NaOH added. After dissolution of the insulin analog 295 $\mu$l 0.1 M ZnCl$_2$ was added and the solution stirred until a temporary precipitate was dissolved. 315 $\mu$l 0.32 mM phenol and 98 $\mu$l 0.5 M glycylglycine and 70 $\mu$l 1 % Tween 20 were subsequently added and pH measured to 7.60. Finally 693 $\mu$l water was added and the solution was passed through a sterile 0.22 $\mu$m Millex®-GV filter unit to obtain 7 ml 9 mM B29-N$^\varepsilon$-(N-lithocholyl-$\gamma$-glutamyl)-des(B30) human insulin. The solution remained stable after 3 months at 5 °C.

Table 1

| Stability of solutions of human insulin at conventional phenol/cresol concentrations (used for multiple dose containers) as a function of salt concentration, ion charge, and detergent concentration. | |
| --- | --- |
| **Excipient** | **Physical stability of solution at 5 °C** |
| 0.5 Zn$^{2+}$ /insulin phenol and cresol 16 mM pH 7.5 and added (mM): | Maximal concentration without precipitation for 4 months. Test solutions were 0.6, 3, 6, 9, 12 and 15 mM insulin, respectively. |
| reference (norm. dissolution[*]) | 3 - 6 |
| reference (low ion strength) | 12 |
| NaCl 10 | 15 |
| NaCl 20 | 12 |
| NaCl 40 | 6 |
| NaCl 60 | <3 |
| NaH$_2$PO$_4$ 5 | |
| + NaCl 20 | 6 |
| + NaCl 25 | 6 |
| + NaCl 37.5 | 6 |
| + NaCl 50 | 6 |
| glycylglycine 7 | 15 |
| glycylglycine 12 | 15 |
| glycylglycine 24 | 15 |
| glycylglycine 48 | 15 |
| glycylglycine 72 | 15 |
| glycylglycine 96 | 15 |
| glycylglycine 120 | 15 |
| glycine 10 | 15 |
| glycine 20 | 15 |
| glycine 40 | 15 |
| glycine 60 | 15 |
| glycine 80 | 15 |
| glycine 100 | 15 |
| trishydroxymethylaminomethan[**]7 | 12 |
| tris 12 | 9 |
| tris 24 | 9 |
| tris 48 | 3 |
| tween 20 0.05 % | 15 |

[*] addition of 1 $\mu$l 1N hydrochloric acid per mg insulin corresponding to about 6 equivalents of chloride.

[**] neutralised by hydrochloric acid

Table 1   (continued)

| Excipient | Physical stability of solution at 5 °C |
|---|---|
| Stability of solutions of human insulin at conventional phenol/cresol concentrations (used for multiple dose containers) as a function of salt concentration, ion charge, and detergent concentration. | |
| 0.5 $Zn^{2+}$ /insulin phenol and cresol 16 mM pH 7.5 and added (mM): | Maximal concentration without precipitation for 4 months. Test solutions were 0.6, 3, 6, 9, 12 and 15 mM insulin, respectively. |
| tween 20 0.2 % | 15 |
| tween 20 1 % | 15 |
| tween 20 5 % | <3 |
| Polox 188 0.2% | 12 |
| Polox 188 1 % | 12 |
| sodium taurocholate 3 | 12 |
| sodium taurocholate 15 | 9 |

Table 2

| Excipient | Physical stability of solution at 5 °C | Chemical stability at 37 °C | |
|---|---|---|---|
| Stability of human insulin at equimolar concentrations of phenolic preservatives. | | | |
| 0.5 $Zn^{2+}$/insulin, NaCl 15 mM, glycylglycine 7 mM, tween20 0.01%, pH 7.5 and equimolar: | Maximal stable concentration without precipitation for 3 months at 3, 6, 9, 12, 15 mM insulin | % polymer / week 3 and 15 mM insulin | |
| cresol | 15 | 0.55 | 0.56 |
| phenol | 15 | 0.37 | 0.39 |
| chlor-cresol | 15 | 0.51 | 0.40 |
| thymol | 9 | 0.85 | 1.25 |
| reference (without phenolics) | 6 | 0.94 | 1.49 |

Table 3

| Excipient | | Chemical stability at 37 °C | |
|---|---|---|---|
| Stability of human insulin at varied concentrations of phenolic preservatives. | | | |
| 0.5 $Zn^{2+}$/insulin, NaCl 15 mM, glycylglycine 7 mM, tween20 0.01%, pH 7.5 and | Equivalent phenolic compound per insulin molecule | % polymer / week 3 and 9 mM insulin | |
| reference | 0 | 0.99 | 1.43 |
| phenol | 0.33 | 0.69 | 0.96 |
| phenol | 0.67 | 0.52 | 0.55 |
| phenol | 1 | 0.46 | 0.38 |
| phenol | 2 and 1.33 | 0.27 | 0.26 |
| chloro-cresol | 0.33 | 0.66 | 0.93 |

Table 3   (continued)

| Stability of human insulin at varied concentrations of phenolic preservatives. | | | |
|---|---|---|---|
| **Excipient** | | **Chemical stability at 37 °C** | |
| 0.5 Zn$^{2+}$/insulin, NaCl 15 mM, glycylglycine 7 mM, tween20 0.01%, pH 7.5 and | Equivalent phenolic compound per insulin molecule | % polymer / week | |
| chloro-cresol | 0.67 | 0.48 | 0.58 |
| chloro-cresol | 1 | 0.30 | 0.30 |
| chloro-cresol | 2 and 1.33 | 0.13 | 0.18 |

**Claims**

1. An aqueous insulin formulation comprising: 6 to 15 mM of human insulin or an analogue or a derivative thereof, less than 50 mM of chloride, less than 10 mM of any anions other than chloride and acetate, 2 to 5 Zn$^{2+}$ ions per six molecules of insulin and at least 3 phenolic molecules per six molecules of insulin.

2. An insulin formulation according to claim 1 comprising about 6 mM, about 9 mM, about 12 mM, or about 15 mM of human insulin or an analogue or a derivative thereof.

3. An insulin formulation according to claim 1 or 2 comprising up to 50 mM of phenolic molecules, preferably 3 to 12 phenolic molecules per six molecules of insulin, more preferably 3 to 9 phenolic molecules per six molecules of insulin.

4. An insulin formulation according to any of the preceding claims comprising less than 40 mM, preferably less than 30 mM of chloride, more preferably 5 to 20 mM of chloride.

5. An insulin formulation according to any of the preceding claims comprising up to 5 mM of phosphate.

6. An insulin formulation according to any of the preceding claims comprising 2 to 4 Zn$^{2+}$ ions, preferably 2.2 to 3.2 Zn$^{2+}$ ions per six molecules of insulin.

7. An insulin formulation according to any of the preceding claims, further comprising 5 to 150 mM of a zwitterionic amine, preferably glycylglycine or glycine.

8. An insulin formulation according to any of the preceding claims, further comprising 5 to 50 mM of trishydroxymethylaminomethan.

9. An insulin formulation according to any of the preceding claims comprising sodium ions, potassium ions, or a mixture thereof, as cations.

10. An insulin formulation according to any of the preceding claims, further comprising between 0.001 % by weight and 1 % by weight of a non-ionic surfactant, preferably tween 20 or Polox 188.

11. An insulin formulation according to any of the preceding claims, further comprising 1 mM to 10 mM of an anionic surfactant, preferably sodium taurocholate.

12. An insulin formulation according to any of the preceding claims comprising human insulin.

13. An insulin preparation according to any of the preceding claims, comprising an analogue of human insulin wherein position B28 Is Asp, Lys, Leu, Val or Ala and position B29 is Lys or Pro; or des(B28-B30), des(B27) or des(B30) human insulin.

14. An insulin preparation according to claim 13, comprising an analogue of human insulin wherein position B28 is Asp or Lys, and position B29 is Lys or Pro, preferably Asp$^{B28}$ human insulin or Lys$^{B28}$Pro$^{B29}$ human insulin.

15. An insulin preparation according to any one of the claims 1 to 11, comprising a derivative of human insulin having one or more lipophilic substituents, preferably an acylated insulin.

16. An insulin preparation according to claim 15, wherein the insulin derivative is selected from the group consisting of B29-N$^\varepsilon$-myristoyl-des(B30) human insulin, B29-N$^\varepsilon$-palmitoyl-des(B30) human insulin, B29-N$^\varepsilon$-myristoyl human insulin, B29-N$^\varepsilon$-palmitoyl human insulin, B28-N$^\varepsilon$-myristoyl Lys$^{B28}$Pro$^{B29}$ human insulin, B28-N$^\varepsilon$-palmitoyl Lys$^{B28}$ Pro$^{B29}$ human insulin, B30-N$^\varepsilon$-myristoyl-Thr$^{B29}$Lys$^{B30}$ human insulin, B30-N$^\varepsilon$-palmitoyl-Thr$^{B29}$Lys$^{B30}$ human insulin, B29-N$^\varepsilon$-(N-paimitoyl-$\gamma$-glutamyl)-des(B30) human insulin, B29-N$^\varepsilon$-(N-lithocholyl-$\gamma$-glutamyl)-des(B30) human insulin, B29-N$^\varepsilon$-($\omega$-carboxyheptadecanoyl)-des(B30) human insulin and B29-N$^\varepsilon$-($\omega$-carboxyheptadecanoyl) human insulin.

17. An insulin preparation according to claim 16, wherein the insulin derivative is B29-N$^\varepsilon$myristoyl-des(B30) human insulin or B29-N$^\varepsilon$-(N-lithocholyl-$\gamma$-glutamyl)-des(B30) human insulin.

18. An insulin preparation according to any one of the preceding claims, comprising an insulin analogue or human insulin as well as an insulin derivative.

19. An insulin preparation according to any one of the preceding claims, wherein the phenolic molecules are selected from the group consisting of phenol, m-cresol, chloro-cresol, thymol, or any mixture thereof.

20. An insulin preparation according to any one of the preceding claims having a pH value in the range of 7 to 8.5, preferably 7.4 to 7.9.

**Patentansprüche**

1. Wässrige Insulin-Formulierung, enthaltend: 6 bis 15 mM humanes Insulin oder ein Analogon oder ein Derivat hiervon, weniger als 50 mM Chlorid, weniger als 10 mM eines beliebigen Anions, mit Ausnahme von Chlorid und Azetat, 2 bis 5 Zn$^{2+}$-Ionen je 6 Insulin-Moleküle und mindestens 3 Phenol-Moleküle je 6 Insulin-Moleküle.

2. Insulin-Formulierung nach Anspruch 1, enthaltend ca. 6 mM, ca. 9 mM, ca. 12 mM oder ca. 15 mM humanes Insulin oder ein Analogon oder ein Derivat hiervon.

3. Insulin-Formulierung nach Anspruch 1 oder 2, enthaltend bis zu 50 mM Phenol-Moleküle, bevorzugt 3 bis 12 Phenol-Moleküle je 6 Insulin-Moleküle, besonders bevorzugt 3 bis 9 Phenol-Moleküle je 6 Insulin-Moleküle.

4. Insulin-Formulierung nach einem der vorangehenden Ansprüche, enthaltend weniger als 40 mM, bevorzugt weniger als 30 mM Chlorid, besonders bevorzugt 5 bis 20 mM Chlorid.

5. Insulin-Formulierung nach einem der vorangehenden Ansprüche, enthaltend bis zu 5 mM Phosphat.

6. Insulin-Formulierung nach einem der vorangehenden Ansprüche, enthaltend 2 bis 4 Zn$^{2+}$-Ionen, bevorzugt 2,2 bis 3,2 Zn$^{2+}$-Ionen je 6 Insulin-Moleküle.

7. Insulin-Formulierung nach einem der vorangehenden Ansprüche, ferner enthaltend 5 bis 150 mM eines zwitterionschen Amins, bevorzugt Glycylglycin oder Glycin.

8. Insulin-Formulierung nach einem der vorangehenden Ansprüche, ferner enthaltend 5 bis 50 mM Trishydroxymethylaminomethan.

9. Insulin-Formulierung nach einem der vorangehenden Ansprüche, enthaltend Natriumionen, Kaliumionen oder eine Mischung hiervon, als Kationen.

10. Insulin-Formulierung nach einem der vorangehenden Ansprüche, ferner enthaltend zwischen 0,001 Gewichts-% und 1 Gewichts-% eines nicht-ionischen oberflächenaktiven Stoffes, bevorzugt Tween 20 oder Polox 188.

11. Insulin-Formulierung nach einem der vorangehenden Ansprüche, ferner enthaltend 1 mM bis 10 mM eines anionischen oberflächenaktiven Stoffes, bevorzugt Natriumtaurocholat.

**12.** Insulin-Formulierung nach einem der vorangehenden Ansprüche, enthaltend humanes Insulin.

**13.** Insulin-Zubereitung nach einem der vorangehenden Ansprüche, enthaltend ein Analogon von humanem Insulin, worin die Position B28 Asp, Lys, Leu, Val oder Ala ist und die Position B29 Lys oder Pro ist; oder des(B28-B30), des(B27) oder des(B30) humanes Insulin ist.

**14.** Insulin-Zubereitung nach Anspruch 13, enthaltend ein Analogon von humanem Insulin, worin die Position B28 Asp oder Lys ist und die Position B29 Lys oder Pro ist, bevorzugt Asp$^{B28}$ humanes Insulin oder Lys$^{B28}$Pro$^{B29}$ humanes Insulin.

**15.** Insulin-Zubereitung nach einem der Ansprüche 1 bis 11. enthaltend ein Derivat von menschlichem Insulin mit einem oder mehreren lipophilen Substituenten, bevorzugt ein acyliertes Insulin.

**16.** Insulin-Zubereitung nach Anspruch 15, worin das Insulin-Derivat ausgewählt ist aus der Gruppe bestehend aus B29-N$^\varepsilon$-Myristoyl-des(B30) humanem Insulin, B29-N$^\varepsilon$-Palmitoyl-des(B30) humanem Insulin, B29-N$^\varepsilon$-Myristoyl humanem Insulin, B29-N$^\varepsilon$-Palmitoyl humanem Insulin, B28-N$^\varepsilon$-Myristoyl Lys$^{B28}$ Pro$^{B29}$ humanem Insulin, B28-N$^\varepsilon$-Palmitoyl Lys$^{B28}$ Pro$^{B29}$ humanem Insulin, B30-N$^\varepsilon$-Myristoyl-Thr$^{B29}$ Lys$^{B30}$ humanem Insulin, B30-N$^\varepsilon$-Palmitoyl-Thr$^{B29}$ Lys$^{B30}$ humanem Insulin, B29-N$^\varepsilon$-(N-Palmitoyl-γ-glutamyl)-des(B30) humanem Insulin, B29-N$^\varepsilon$-(N-Lithocholyl-γ-glutamyl)-des(B30) humanem Insulin, B29-N$^\varepsilon$-(ω-Carboxyheptadecanoyl)-des(B30) humanem Insulin und B29-N$^\varepsilon$-(ω-Carboxyheptadecanoyl) humanem Insulin.

**17.** Insulin-Zubereitung nach Anspruch 16, worin das Insulinderivat B29-N$^\varepsilon$-Myristoyl-des(B30) humanes Insulin oder B29-N$^\varepsilon$-(N-Lithocholyl-γ-glutamyl)-des(B30) humanes Insulin ist.

**18.** Insulin-Zubereitung nach einem der vorangehenden Ansprüche, enthaltend ein Insulin-Analogon oder humanes Insulin sowie ein Insulin-Derivat.

**19.** Insulin-Zubereitung nach einem der vorangehenden Ansprüche, worin die Phenol-Moleküle ausgewählt sind aus der Gruppe bestehend aus Phenol, m-Kresol, chloro-Kresol, Thymol oder eine Mischung hiervon.

**20.** Insulin-Zubereitung nach einem der vorangehenden Ansprüche mit einem pH-Wert in dem Bereich von 7 bis 8,5, bevorzugt 7,4 bis 7,9.

**Revendications**

**1.** Formulation d'insuline aqueuse comportant : 6 à 15 mM d'insuline humaine ou d'un analogue ou d'un dérivé de celle-ci,moins de 50 mM de chlorure, moins de 10 mM de tout anion autre que du chlorure et de l'acétate, 2 à 5 ions Zn$^{2+}$ pour six molécules d'insuline et au moins 3 molécules phénoliques pour six molécules d'insuline.

**2.** Formulation d'insuline selon la revendication 1, comportant environ 6 mM, environ 9 mM, environ 12 mM, ou environ 15 mM d'insuline humaine ou d'un analogue ou d'un dérivé de celle-ci.

**3.** Formulation d'insuline selon la revendication 1 ou 2, comportant jusqu'à 50 mM de molécules phénoliques, de préférence de 3 à 12 molécules phénoliques pour six molécules d'insuline, de manière plus préférée de 3 à 9 molécules phénoliques pour six molécules d'insuline.

**4.** Formulation d'insuline selon l'une quelconque des revendications précédentes, comportant moins de 40 mM, de préférence moins de 30 mM de chlorure, de manière plus préférée de 5 à 20 mM de chlorure.

**5.** Formulation d'insuline selon l'une quelconque des revendications précédentes, comportant jusqu'à 5 mM de phosphate.

**6.** Formulation d'insuline selon l'une quelconque des revendications précédentes, comportant 2 à 4 ions Zn$^{2+}$, de préférence 2,2 à 3,2 ions Zn$^{2+}$, pour six molécules d'insuline.

**7.** Formulation d'insuline selon l'une quelconque des revendications précédentes, comportant de plus 5 à 150 mM d'une amine dipolaire, de préférence une glycylglycine ou une glycine.

**8.** Formulation d'insuline selon l'une quelconque des revendications précédentes, comportant de plus 5 à 50 mM de trishydroxyméthylaminométhane.

**9.** Formulation d'insuline selon l'une quelconque des revendications précédentes, comportant des ions sodium, des ions potassium ou un mélange de ceux-ci, en tant que cations.

**10.** Formulation d'insuline selon l'une quelconque des revendications précédentes, comportant de plus entre 0,001 % en poids et 1 % en poids d'un tensioactif non-ionique, de préférence tween 20 ou Polox 188.

**11.** Formulation d'insuline selon l'une quelconque des revendications précédentes, comportant de plus de 1 mM à 10 mM d'un tensioactif anionique, de préférence du taurocholate de sodium.

**12.** Formulation d'insuline selon l'une quelconque des revendications précédentes, comportant de l'insuline humaine.

**13.** Préparation d'insuline selon l'une quelconque des revendications précédentes, comportant un analogue d'insuline humaine dans lequel la position B28 est Asp, Lys, Leu, Val ou Ala et la position B29 est Lys ou Pro, ou de l'insuline humaine des(B28-B30), des(B27) ou des(B30).

**14.** Préparation d'insuline selon la revendication 13, comportant un analogue d'insuline humaine dans lequel la position B28 est Asp ou Lys, et la position B29 est Lys ou Pro, de préférence une insuline humaine Asp$^{B28}$ ou une insuline humaine Lys$^{B28}$Pro$^{B29}$.

**15.** Préparation d'insuline selon l'une quelconque des revendications 1 à 11, comportant un dérivé d'insuline humaine ayant un ou plusieurs substituants lipophiles, de préférence une insuline acylée.

**16.** Préparation d'insuline selon la revendication 15, dans laquelle le dérivé d'insuline est sélectionné parmi le groupe constitué d'insuline humaine B29-N$^\varepsilon$-myristoyle-des(B30), d'insuline humaine B29-N$^\varepsilon$-palmitoyle-des(B30), d'insuline humaine B29-N$^\varepsilon$-myristoyle, d'insuline humaine B29-N$^\varepsilon$-palmitoyle, d'insuline humaine B28-N$^\varepsilon$-myristoyle-Lys$^{B28}$Pro$^{B29}$, d'insuline humaine B28-N$^\varepsilon$-palmitoyle-Lys$^{B28}$Pro$^{B29}$, d'insuline humaine B30-N$^\varepsilon$- myristoyle-Thr$^{B29}$Lys$^{B30}$, d'insuline humaine B30-N$^\varepsilon$-palmitoyle-Thr$^{B29}$Lys$^{B30}$, d'insuline humaine B29-N$^\varepsilon$-(N-palmitoyle-$\gamma$-glutamyle)-des(B30), d'insuline humaine B29-N$^\varepsilon$-(N-lithocholyle-$\gamma$-glutamyle)-des(B30), d'insuline humaine B29-N$^\varepsilon$-($\omega$-carboxyheptadécanoyle)-des(B30) et d'insuline humaine B29-N$^\varepsilon$-($\omega$-carboxyheptadécanoyle).

**17.** Préparation d'insuline selon la revendication 16, dans laquelle le dérivé d'insuline est l'insuline humaine B29-N$^\varepsilon$-myristoyle-des(B30) ou l'insuline humaine B29-N$^\varepsilon$-(N-lithocholyle-$\gamma$-glutamyle) -des (B30).

**18.** Préparation d'insuline selon l'une quelconque des revendications précédentes, comportant un analogue d'insuline ou une insuline humaine ainsi qu'un dérivé d'insuline.

**19.** Préparation d'insuline selon l'une quelconque des revendications précédentes, dans laquelle les molécules phénoliques sont sélectionnées parmi le groupe constitué de phénol, m-crésol, chlorocrésol, thymol, ou tout mélange de ceux-ci.

**20.** Préparation d'insuline selon l'une quelconque des revendications précédentes, ayant une valeur de pH dans la plage allant de 7 à 8,5, de préférence de 7,4 à 7,9.